# EUROPEAN PATENT SPECIFICATION

(11) **EP 0 892 649 B1**
(45) Date of publication and mention of the grant of the patent: **23.06.2004**
(21) Application number: 97907219.6
(22) Date of filing: 17.03.1997
(51) Int. Cl.: A61M 5/30

(54) **NEEDLELESS SYRINGE WITH THERAPEUTIC AGENT PARTICLES ENTRAINED IN SUPERSONIC GAS FLOW**
NADELLOSE SPRITZE MIT IN ÜBERSCHALLGASSTRÖMUNG GETRAGENEN THERAPIEWIRKSTOFFPARTIKELN
SERINGUE SANS AIGUILLE DANS LAQUELLE LES PARTICULES D'AGENT THERAPEUTIQUE SONT ENTRAINEES DANS UN FLUX SUPERSONIQUE DE GAZ

(30) Priority: 19.03.1996 GB 9605690
(43) Date of publication of application: 27.01.1999
(73) Proprietor: PowderJect Research Limited, Oxford OX4 4GA (GB)
(72) Inventor: BELLHOUSE, Brian John, Islip, Oxfordshire OX5 2SQ (GB); POTTER, Charles David Ogilvy, Oxon OX8 7SG (GB); GREENFORD, John Christopher, Abingdon, Oxfordshire OX14 1DF (GB)
(74) Representative: Price, Nigel John King
(86) International application number: PCT/GB1997/000734
(87) International publication number: WO 1997/034652

(56) References cited:
- WO-A-94/24263

## Description

The invention is related to a needleless syringe which consists of a tubular nozzle through which there is established a gas flow, which is usually supersonic, and in which particles of a powdered therapeutic agent are entrained. Such a syringe provides a non-invasive drug delivery system for firing controlled doses into body tissue, e.g. through the intact skin. Such a needleless syringe is known from WO-A-9424263. In particular,such a syringe comprises all the features recited in the preamble of claim 1.

The mechanics at the upstream end of the nozzle may involve a reservoir of gas such as helium, at a high pressure of say between 30 and 80 bar, the flow being created by releasing the gas suddenly from the reservoir, for example by opening a valve, or by releasing the gas from the reservoir so that pressure builds up behind a membrane at the entrance to the nozzle until the membrane eventually ruptures to release the high speed gas flow. The particles may be contained initially within the high pressure gas reservoir or within a cassette from which the particles are released into the gas flow. The passageway through the nozzle itself is preferably convergent/divergent or convergent/cylindrical, with the upstream convergent section shorter than the downstream divergent or cylindrical section.

However, the present invention is particularly concerned with the construction of the syringe at the downstream end of the nozzle. It is desirable to dissipate and silence the shockwave which rebounds off the target and which can be very loud and disturbing to a patient. However, any silencer should readily allow the external dissipation of the gas pressure to avoid syringe recoil and lifting of the syringe away from the target. Furthermore, it is desirable that any small proportion of the particles or particle fragments, which are reflected from the target surface, are contained.

In the past, we have attempted to satisfy these desiderata by providing a tubular shroud having a cylindrical silencer portion which surrounds with a radial spacing a downstream section of the nozzle, and a spacer portion which extends axially beyond the exit plane of the nozzle to abut the target and positively space the exit plane of the nozzle from the target surface. The annular chamber between the nozzle and cylindrical portion contains baffles which create a tortuous path for the gas and, at its end remote from the spacer portion the cylindrical portion is provided with ports for venting the gas to the atmosphere. Such a shroud is illustrated in Figs. 1 to 3 of our WO 94/24263. Although practical, such a shroud is still noisy owing to the comparatively few large vent ports, and it is still possible for some of the particles to pass out of the ports after multiple reflections.

In accordance with the present invention, a needleless syringe comprises an elongate tubular nozzle which is, or is arranged to be, connected at its upstream end to a source of high pressure gas; a source of particles of a powdered therapeutic agent; and means for releasing the gas to provide through the nozzle a supersonic flow in which the particles are entrained; wherein the downstream end of the nozzle opens into a tubular shroud which is arranged to engage a target and hence to space the exit plane of the nozzle from the target surface and which comprises a porous material the porosity of which is such as to contain within the shroud any particles reflected from the target but to allow passage of the gas out of the shroud through the porous material and thereby reduce the noise of the shockwave.

The use of a porous material, having an appropriate porosity, positively contains within the shroud all the particles which may have been reflected from the target while allowing the gas to pass out of the shroud through the material and thereby providing very effective silencing. This may be enhanced by providing baffles within the shroud, particularly in an annular chamber between at least a downstream section of the nozzle and a surrounding tubular portion of the shroud, or provided by steps in the external wall of the nozzle and/or the internal wall of the shroud.

The porous material could be an outer sleeve surrounding a solid shroud having comparatively few large vents.

Alternatively a tubular outer wall of the shroud is formed partly or substantially completely of the porous material. An appropriate porous material for the shroud may be a sintered porous media, manufactured by dry powder moulding of particles of, for example, polyethylene, polypropylene or a mixture thereof, or even of metals such as stainless steel or bronze.

The material could have more than one porosity, eg a coarse pore size inner layer and a fine pore size outer layer or vice versa, or even a graded pore size from inside to outside. The finer material will catch any stray particles, whereas it is believed that the variable pore size will successively and more efficiently, break up the shockwave.

As a further option, an outer substantially rigid tubular wall of the shroud may be provided with one or more gas exit openings and the porous material is a wadding, such as cotton wool, closing the upstream end(s) of the opening(s).

The porosity needs to be carefully chosen and this depends on the range of particles sizes used. Too large a porosity will enable some stray particles to pass through the material to the atmosphere. Too small a porosity or too thick a material will not readily allow the gas to pass through, thus causing a high pressure at the tip of the syringe and therefore cause unwanted recoil. Experiments with a drug having a mean particle size of 30 microns have successfully been carried out using a shroud made of an open pore solid material which has a mean pore size of 15 microns, a minimum pore size of 4 microns, and with a porosity which is 55% solid and 45% void. The wall thickness was 3mm.

Examples of syringes constructed in accordance with the present invention are illustrated in the accompanying drawings, in which:
Fig. 1 is an axial section through one syringe;
Fig. 2 is an axial section through the downstream end of another syringe;
Fig. 3 is a half axial section of a third syringe; and,
Fig. 4 is an enlargement of part of Fig. 3

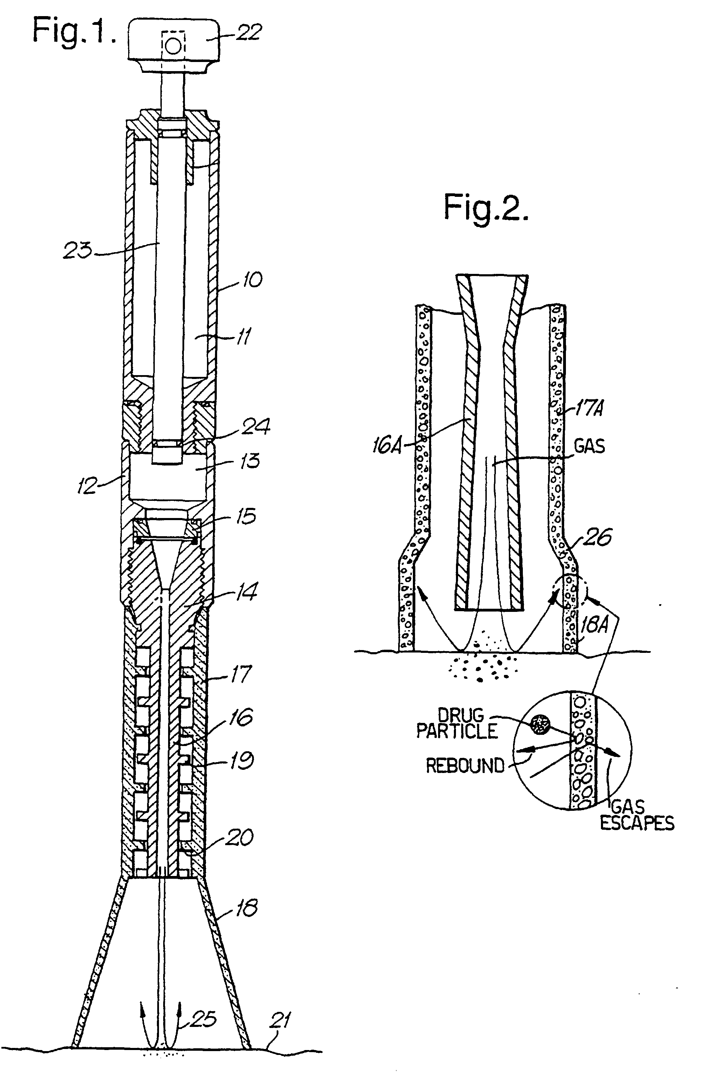

The syringe illustrated in Fig. 1 is similar in many respects to that illustrated in Figs. 1 to 3 of WO 94/24263. Thus it has an upper barrel portion 10 providing a reservoir 11 of compressed gas, such as helium at between 30 and 80 bar. The upper barrel portion 10 is screwed to a lower barrel portion 12 providing a rupture chamber 13. The lower barrel portion 12 is screwed to the. upper end of a nozzle 14 having a convergent/divergent passageway. Sealed between the lower barrel portion 12 and the upper end of the nozzle 14 is a cassette 15 consisting of upper and lower rupturable membranes between which is sealed a dose of a powdered therapeutic agent.

A lower part 16 of the nozzle is surrounded by a shroud comprising a cylindrical portion 17 and a flared portion 18. The nozzle portion 16 has external annular ribs 19 which interdigitate with internal annular ribs 20 within the shroud portion 17.

In use the wider end of the shroud portion 18 is brought into contact with the skin 21 of a patient and a knob 22 is depressed. This causes a valve stem 23 to move through the reservoir 11 so that a seal 24 moves out of an exit passageway of the reservoir and releases gas from the reservoir into the rupture chamber 13. As the pressure builds up, it eventually ruptures the membranes of the cassette 15 releasing a supersonic gas flow through the nozzle with the particles of therapeutic agent entrained within the flow. These particles penetrate the patient's skin 21 to provide the necessary transdermal injection. The shroud portion 18 acts as a spacer which positively spaces the exit plane of the nozzle from the patient's skin and allows a degree of spread in the particles as they pass from the nozzle to the target. The shockwave resulting from the gas flow rebounds from the patient's skin, together with a small proportion of the particles, as suggested by the arrows 25 whereupon the pressure and noise of the shockwave are dissipated and the particles are caught within the shroud.

This dissipation and containment are efficiently achieved by constructing the shroud of an open pore material as implied by the enlargement in the circle in Fig. 1. The shockwave is not only dissipated by the tortuous path between the baffles formed by the interdigitating ribs 19 and 20, but also by passage through the porous outer wall of the shroud. Since at least the majority of the shroud is made as such porous material, there is a minimum resistance to outflow of the gas through the shroud wall, thereby minimising any recoil of the syringe away from the patient's skin. However, the pore size is sufficiently small to retain any particles, or particle fragments, which may rebound.

The shroud may be moulded in complementary halves, divided by an axial plane, so as to be fitted around the nozzle and between the ribs 19 upon assembly with the nozzle, prior to the halves being bonded together. The upper end of the shroud portion 17 will be bonded, snap fitted, or otherwise secured to the upper portion 14 of the nozzle.

Fig. 2 shows a modification in which the baffles provided by the ribs 19 and 20 are omitted but the shroud is provided with a step 26 between its smaller and larger diameter portions 17A and 18A surrounding the nozzle 16A. This step acts to some degree in providing adequate rebound surfaces to initiate dissipation of the shockwave and deceleration of any rebounding particles.

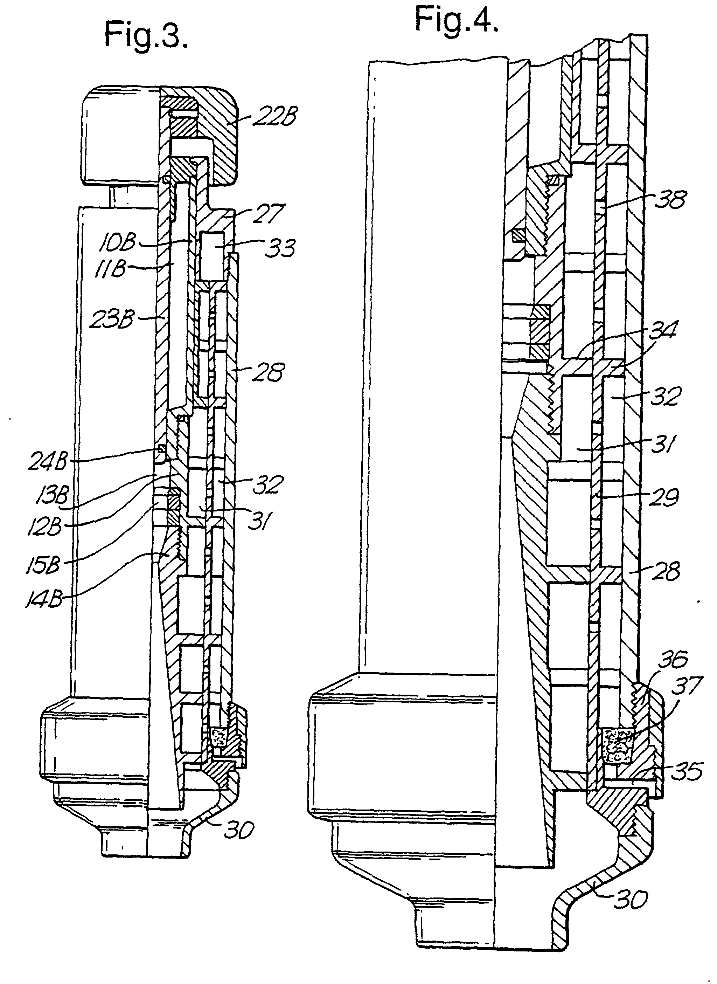

The syringe shown in Figs. 3 and 4 has a particle delivery mechanism which is similar to that of the Fig. 1 syringe. Parts of the third syringe, which, although of a different detailed shape, have the same function as parts 10-15 and 22-24 in Fig. 1 are given the reference numerals 10B-15B and 22B-24B. Further description of these parts is therefore deemed unnecessary.

The essential difference between the third example and first example of syringe lies in the construction and operation of the tubular shroud. In the third example this extends up almost to the top of the reservoir 11B and comprises a number of plastic parts which are welded or screwed together. These parts include an upper part 27, which is hung on the top of the upper barrel portion 10B, an outer cylindrical wall 28, an intermediate cylindrical wall 29 and an outlet portion 30, into which the downstream end of the passageway through the nozzle 14B opens. The portion 30 is shown as having a generally frustoconical portion merging into a substantially cylindrical portion. This section acts as a spacer which, when pressed against the target surface, positively spaces the downstream of the nozzle a distance of about 10 mm away from the target surface.

The intermediate cylindrical wall 29 divides the annular space between the outer wall 28 and the internal parts of the syringe into two coaxial radially inner and outer annular chambers 31 and 32. These chambers are in series, being interconnected by a hollow interior 33 of the part 27, and each provides a tortuous path by virtue of discontinuous baffles 34. In each chamber 31, 32, the baffles are axially spaced about 10 mm apart and each consists of an annular disc with two diametrically opposed slots, each occupying about 60° of arc, with the slots in one baffle 90° out of alignment with those in the adjacent baffle in the same chamber. The lower end, as shown in Figs. 3 and 4, of the chamber 31 is open to the interior of the section 30. The lower end of the outer chamber 32 communicates with a ring of twenty four exit openings 35, in a nut member 36, each opening being provided by a bore having a diameter of about 2 mm. Located between the wall 28 and the nut member 36 is an annular ring of cotton-wool wadding 37 which isolates the interior of the chamber 32 from the openings 35.

In use the gas and any particles reflected from the target surface are caused to pass up the chamber 31, following a tortuous path around the baffles 34, and hence via the chamber 33 into the chamber 32, where they pass down again along a tortuous path around the baffles 34. The gas is able to permeate through the body 37 and hence be vented to atmosphere through the openings 35 while the wadding 37 captures any residual particles.

The larger the volume of the chambers 31 and 32 the better. However, it has been found in practice that a vented volume of about 80 ml works very well.

The function of the silencer is enhanced if there is provision for leakage of gas from the chamber 31 to the chamber 32 through the wall 29. This may be provided by perforations 38 in the wall 29 and an appropriate arrangement is four equalangularly spaced holes through the wall between each pair of baffles 34. Those holes further away from the target, ie higher up in Figs. 3 and 4, are preferably a little larger than those lower down. For example, holes in the upper half may be 2 mm in diameter and those in the lower half 1.5 mm in diameter. As an alternative to the holes 38, the wall 29 could be made of an open pore material.

Experiments show that the construction shown in Figs. 3 and 4 enables the noise level to be reduced to an acceptable level of below 80 dB at 1m distance with an applied load of 20N.

## Claims

1. A needleless syringe comprising an elongate tubular nozzle (14) which is, or is arranged to be, connected at its upstream end to a source (11) of high pressure gas; a source (15) of particles of a powdered therapeutic agent; and means for releasing the gas to provide through the nozzle a supersonic flow in which the particles are entrained; wherein the downstream end of the nozzle opens into a tubular shroud (17). which is arranged to engage a target (21) and hence to space the exit plane of the nozzle from the target surface; said syringe being **characterised in that** said shroud (17) comprises a porous material the porosity of which is such as to contain within the shroud any particles reflected from the target but to allow passage of the gas out of the shroud through the porous material and thereby reduce the noise of the shockwave.

2. A syringe according to claim 1, wherein a tubular outer wall of the shroud is made of a substantially rigid open pore material.

3. A syringe according to claim 1, wherein an outer substantially rigid tubular wall (28,36,30) of the shroud is provided with one or more gas exit openings (35) and the porous material is wadding (37) closing the upstream end(s) of the opening(s).

4. A syringe according to any one of the preceding claims, wherein the shroud has a tubular silencer portion which surrounds with a radial spacing at least a downstream section of the nozzle (14B) to provide between the nozzle and portion an annular chamber (31,32) containing baffles (34) which create a tortuous path for the gas before passing out of the shroud.

5. A syringe according to claim 4, wherein an intermediate tubular portion (31) is located between the downstream section of the nozzle and the silencer portion of the shroud to provide radially inner and outer coaxial annular chambers (31,32) in series containing baffles (34).

6. A syringe according to claim 5, wherein the intermediate tubular portion (31) is porous or perforated (38) to allow gas leakage between the radially inner annular chamber (31) and the radially outer annular chamber (32).

## Patentansprüche

1. Nadellose Spritze, umfassend eine verlängerte röhrenförmige Düse (14), welche an ihrem stromaufwärtigen Ende mit einer Quelle (11) eines unter hohem Druck stehenden Gases verbunden ist oder derart angeordnet ist, um damit verbunden zu werden; eine Quelle (15) von Partikeln eines pulvrigen therapeutischen Mittels; und Mittel zum Freigegeben des Gases, um durch die Düse eine Überschallströmung bereitzustellen, in welcher die Partikel mitgerissen werden; wobei sich das stromabwärtige Ende der Düse in einer röhrenförmige Abdeckung (17) öffnet, welche angeordnet ist, um mit einem Ziel (21) in Eingriff gebracht zu werden und infolgedessen die Ausgangsebene der Düse und die Zieloberfläche mit einem Zwischenraum anzuordnen; wobei die Spritze **dadurch gekennzeichnet ist, dass** die Abdeckung (17) ein poröses Material umfasst, dessen Porosität derart ist, dass in der Abdeckung jegliche Partikel, welche von dem Ziel reflektiert werden, erfasst werden, aber dass ein Durchgang des Gases aus der Abdeckung durch das poröse Material ermöglicht und dadurch das Geräusch der Schockwelle vermindert wird.

2. Spritze nach Anspruch 1, wobei eine röhrenförmige Außenwand der Abdeckung aus einem im Wesentlichen steifen offenporigen Material hergestellt ist.

3. Spritze nach Anspruch 1, wobei eine äußere im Wesentlichen steife röhrenförmige Wand (28, 36, 30) der Abdeckung mit einer oder mehreren Gasausgangsöffnungen (35) versehen ist und das poröse Material ein Füllmaterial (37) ist, welches das(die) stromaufwärtige(n) Ende(n) der Öffnung(en) verschließt.

4. Spritze nach einem der vorhergehenden Ansprüche, wobei die Abdeckung einen röhrenförmigen Dämpferabschnitt aufweist, welcher mit einem radialen Abstand mindestens eine stromabwärtige Sektion der Düse (14B) umgibt, um zwischen der Düse und dem Abschnitt eine ringförmige Kammer (31, 32) bereitzustellen, welche Umlenkungen (34) enthält, die einen gewundenen Pfad für das Gas erzeugen, bevor es die Abdeckung passiert.

5. Spritze nach Anspruch 4, wobei ein röhrenförmiger Zwischenabschnitt (31) zwischen der stromabwärtigen Sektion der Düse und dem Dämpferabschnitt der Abdeckung angeordnet ist, um innere und äußere koaxiale ringförmige Kammern (31, 32) in Reihe bereitzustellen, welche Umlenkungen (34) enthalten.

6. Spritze nach Anspruch 5, wobei der röhrenförmige Zwischenabschnitt (31) porös oder perforiert (38) ist, um einen Gasabfluss zwischen der radialen inneren ringförmigen Kammer (31) und der radialen äußeren ringförmigen Kammer (32) zu ermöglichen.

## Revendications

1. Seringue sans aiguille comprenant une buse tubulaire allongée (14) qui est, ou qui est configurée de façon à être, reliée à son extrémité amont à une source (11) de gaz à haute pression ; une source (15) de particules d'un agent thérapeutique en poudre ; et des moyens pour libérer le gaz de façon à produire à travers la buse un écoulement supersonique dans lequel les particules sont entraînées ; dans laquelle l'extrémité aval de la buse s'ouvre dans une enveloppe tubulaire (17) qui est configurée de façon à venir en contact avec une cible (21), et, par conséquent, de façon à espacer le plan de sortie de la buse de la surface de la cible ; ladite seringue étant **caractérisée en ce que** ladite enveloppe (17) comprend un matériau poreux dont la porosité est telle qu'il maintienne à l'intérieur de l'enveloppe toutes les particules réfléchies à partir de la cible, mais qu'il permette le passage du gaz en dehors de l'enveloppe à travers le matériau poreux, et réduise par conséquent le bruit de l'onde de choc.

2. Seringue selon la revendication 1, dans laquelle une paroi extérieure tubulaire de l'enveloppe est réalisée en un matériau à pores ouverts sensiblement rigide.

3. Seringue selon la revendication 1, dans laquelle une paroi tubulaire extérieure sensiblement rigide (28, 36, 30) de l'enveloppe est munie d'une ou plusieurs ouvertures de sortie de gaz (35) et le matériau poreux est une bourre (37) fermant la ou les extrémité(s) amont de la ou des ouverture(s).

4. Seringue selon l'une quelconque des revendications précédentes, dans laquelle l'enveloppe comporte une partie de silencieux tubulaire qui entoure avec un espacement radial au moins une section aval de la buse (14B) afin de constituer entre la buse et la partie une chambre annulaire (31, 32) contenant des écrans (34) qui créent un trajet tortueux pour le gaz avant de sortir de l'enveloppe.

5. Seringue selon la revendication 4, dans laquelle une partie tubulaire intermédiaire (31) est disposée entre la section aval de la buse et la partie de silencieux de l'enveloppe afin de constituer des chambres annulaires coaxiales radialement intérieure et extérieure (31, 32) en série contenant des écrans (34).

6. Seringue selon la revendication 5, dans laquelle la partie tubulaire intermédiaire (31) est poreuse ou perforée (38) de façon à permettre une fuite de gaz entre la chambre annulaire radialement intérieure (31) et la chambre annulaire radialement extérieure (32).
